# EUROPÄISCHE PATENTSCHRIFT

(11) **EP 1 003 702 B1**
(45) Veröffentlichungstag und Bekanntmachung des Hinweises auf die Patenterteilung: **19.06.2002**
(21) Anmeldenummer: 98943743.9
(22) Anmeldetag: 20.07.1998
(51) Int. Cl.: C07C 41/56, C07C 43/303

(54) **VERFAHREN ZUR HERSTELLUNG VON ACETALEN DES 3-METHYL-2-BUTENALS**
METHOD FOR PRODUCING 3-METHYL 2-BUTENAL ACETALS
PROCEDE POUR LA PREPARATION D'ACETALS DU 3-METHYL-2-BUTENAL

(30) Priorität: 01.08.1997 DE 19733258
(43) Veröffentlichungstag der Anmeldung: 31.05.2000
(73) Patentinhaber: BASF AKTIENGESELLSCHAFT, 67056 Ludwigshafen (DE)
(72) Erfinder: HIEBER, Gisela, D-69121 Heidelberg (DE); EBEL, Klaus, D-68623 Lampertheim (DE)
(86) Internationale Anmeldenummer: EP9804479
(87) Internationale Veröffentlichungsnummer: WO9906344

(56) Entgegenhaltungen:
- EP-A- 0 629 619
- DE-A- 2 334 378

## Beschreibung

Die vorliegende Erfindung betrifft ein Verfahren zur Herstellung von Acetalen der Formel I 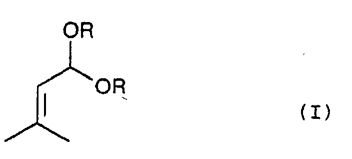 in der
a) R unabhängig voneinander für einen C₁- bis C₂₀-Alkyl- oder C₃bis C₂₀-Alkenylrest stehen oder
b) die beiden Reste R gemeinsam die Glieder eines gegebenenfalls C₁- bis C₁₀-alkylsubstituierten 5 bis 7-gliedrigen cyclischen Acetals bilden,
indem man 3-Methyl-2-butenal in Gegenwart von Amidosulfonsäure, einer N-(C₁- bis C₈-Alkyl-)amidosulfonsäure oder einer N,N-Di(C₁bis C₈-alkyl-)amidosulfonsäure als Katalysator mit
im Fall (a)
a1) einem Alkohol der allgemeinen Formel IIa

   R-OH (IIa)

   in der R die unter (a) genannte Bedeutung hat,
a2) einem Orthoester der allgemeinen Formel (III)

   HC-(OR)₃ (III)

   in der R die unter (a) genannte Bedeutung hat oder
a3) einer Mischung aus einem Alkohol der allgemeinen Formel (IIa) und einem Orthoester der allgemeinen Formel (III),
und im Fall (b)
b) mit einem Alkohol der allgemeinen Formel IIb

   HO-(CH₂)ₘ-OH (IIb)
in der m eine Zahl von 2 bis 4 bedeutet, und eine Methylengruppe im Alkohol IIb durch eine C₁-C₁₀-Alkylgruppe substituiert sein kann, umsetzt.

Die N-(C₁- bis C₈-Alkyl-)amidosulfonsäuren und N,N-Di(C₁- bis C₈-Alkyl-)amidosulfonsäuren werden im folgenden Text kurz "Alkylamidosulfonsäuren" genannt.

Bekannte Verfahren zur Herstellung von Acetalen des 3-Methyl-2-butenals aus 3-Methyl-2-butenal und den entsprechenden Alkoholen lehren den Einsatz verschiedener saurer Katalysatoren . Als Katalysatoren werden üblicherweise folgende Säuren eingesetzt: Phosphorsäure, Ammoniumnitrat, p-Toluolsulfonsäure und Kaliumhydrogensulfat.

Aus Bull. Soc. Fr. **1965,** 1007-1014 ist die Verwendung von Phosphorsäure bekannt. 3-Methyl-2-butenal wird mit Ethanol, Triethylorthoformiat und Phosphorsäure 40 Stunden bei Raumtemperatur gerührt. Die Reaktionsmischung wird dann in Ether aufgenommen, mit 0,5 N wäßrigem Ammoniak gewaschen und über Nacriumsulfat getrocknet. Die anschließende Destillation ergibt 3-Methyl-2-butenal-diethylacetal in einer Ausbeute von 65%.

In Liebigs Ann. Chem. **1986,** 99-113 wird die Umsetzung von 3-Methyl-2-butenal mit Trimethyl- oder Triethylorthoformiat und des entsprechenden Alkohols unter Verwendung von Ammoniumnitrat als Katalysator beschrieben. Nach einer Reaktionszeit von 8 Stunden bei Raumtemperatur wird der ungelöste Katalysator abfiltriert und nach Zusatz von Kaliumcarbonat wird die Reaktionsmischung destilliert. Das Dimethylacetal wird in einer Ausbeute von 63%, das Diethylacetal in einer Ausbeute von 51% isoliert.

Die Verwendung von p-Toluolsulfonsäure ist in J. Chem. Soc. (C) **1971**, 811-816 beschrieben: 3-Methyl-2-butenal, Methanol und Trimethylorthoformiat werden mit p-Toluol-sulfonsäure 24 Stunden bei Raumtemperatur gerührt. Anschließend wird die Reaktionsmischung mit Wasser verdünnt und mit Ether extrahiert. Die organische Phase wird mit Wasser und wäßriger Natriumhydrogencarbonat-Lösung gewaschen, getrocknet und destilliert. Das Destillat enthält noch 10% 3-Methyl-2-butenal. Angaben über die Ausbeute werden nicht gemacht.

In J. Org. Chem. **1995,** *60*, 3397-3400 wurden verschiedene saure Katalysatoren getestet. Die Verwendung von p-Toluolsulfonsäure führte zur Polymerisation des 3-Methyl-2-butenals. Triethylammoniumchlorid, Pyridinium-p-toluolsulfonat, Tetrabutylammoniumhydrogensulfat und Ammoniumhydrogensulfat ergaben ebenfalls keine befriedigenden Resultate. Als besser geeignet erwies sich Kaliumhydrogensulfat. Die Reaktionsbedingungen sind ebenfalls in der Patentanmeldung EP 0629619 beschrieben: Bei 4°C wird abs. Ethanol mit Triethylorthoformiat und 3-Methyl-2-butenal versetzt. Die klare Lösung wird auf 2°C abgekühlt und mit Kaliumhydrogensulfat versetzt. Die heterogene Reaktionsmischung erwärmt sich durch die exotherme Reaktion auf 10°C. Anschließend läßt man während 45 Minuten auf 21°C erwärmen und rührt noch 15 Minuten bei dieser Temperatur. Der Katalysator wird dann abfiltriert und mit Ethanol nachgewaschen. Das Filtrat wird mit Kaliumcarbonat versetzt und eine Stunde bei Raumtemperatur gerührt. Dann wird das Kaliumcarbonat ebenfalls abfiltriert und mit Ethanoi nachgewaschen. Die Destillation ergibt 3-Methyl-2-butenal-dimethylacetal in einer Ausbeute von 85%. Nachteilig an diesem Verfahren ist, daß es einer aufwendigen Temperatursteuerung bedarf und die abwechselnden Kühlungs- und Erwärmungsschritte einen erhöhten Energieverbrauch und eine aufwendige apparative Ausstattung erfordern.

Auch cyclische Acetale, die aufgrund ihrer höheren Stabilität in weitaus besseren Ausbeuten erhalten werden sollten, werden nach dem DE-Patent 2334378 nur in vergleichbaren Ausbeuten wie die offenkettigen Vertreter isoliert (63-88%). Danach werden 3-Methyl-2-butenal und verschiedene 1,3-Diole mit Dichlormethan am Wasserabscheider erhitzt. Als Katalysator wird p-Toluolsulfonsäure verwendet. Überschüssiges 1,3-Diol und p-Toluolsulfonsäure werden nach beendeter Reaktion mit Wasser entfernt. Die organische Phase wird fraktioniert destilliert.

Die Nachteile der bisher bekannten Verfahren unter Verwendung der genannten Säure sind entweder mäßige Ausbeuten, lange Reaktionszeiten oder aufwendige Aufarbeitungsmethoden, häufig infolge der Schwerlöslichkeit des sauren Katalysators. Allen Verfahren ist gemeinsam, daß der saure Katalysator entweder durch einen Filtrationsschritt oder durch eine Wäsche mit Wasser und/oder alkalischen wäßrigen Lösungen aus der Reaktionsmischung entfernt wird.

Die Aufgabe der vorliegenden Erfindung bestand deshalb darin, ein Verfahren bereitzustellen, das die Nachteile des Standes der Technik nicht aufweist und insbesondere die technisch einfache Synthese von Acetalen des 3-Methyl-2-butenals in hohen Ausbeuten ermöglicht.

Demgemäß wurde das eingangs definierte Verfahren gefunden.

Das erfindungsgemäße Verfahren ist im Falle der Herstellung von Verbindungen des Typs (a) der Formel (I) besonders geeignet für solche, die aus C₁- bis C₂₀-Alkyl-Alkoholen wie Methanol, Ethanol, Propanol, i-Propanol, Butanol, sec-Butanol und t-Butanol sowie C₃bis C₂₀-Alkenyl-Alkoholen wie Propenol, Butenol, Pentenol und 3-Methyl-2-butenol, den entsprechenden Orthoestern der allgemeinen Formel (III), deren 3 Alkoxyreste sich von den vcrgenannten Alkoholen ableiten und/oder den Mischungen aus den vorgenannten Alkoholen und Orthoestern gebildet werden.

Falls die Alkohole der Formel (IIa) und die Orthoester der Formel (III) in Form einer Mischung zur Anwendung kommen, kann deren Molverhältnis in einem weiten Bereich variiert werden und beträgt im allgemeinen 0,01 : 1 bis 20 : 1, bevorzugt 0,1 : 1 bis 5 : 1.

Im Falle der Herstellung von Verbindungen der Formel (I) des Typs (b) sind als Alkohole der Formel (IIb), die mit der Carbonylgruppe des 3-Methyl-2-butenals ein gegebenenfalls C₁- bis C₁₀alkylsubsticuiertes Acetal bilden, besonders C₁- bis C₁₀-alkylsubscituierte C₂- bis C₄-Diole wie 1,2-Ethandiol, 1,2-Propandiol, 1,3-Propandiol, 1,2-Butandiol, 1,3-Butandiol, 2,4-Pentandiol, 2-Methyl-2,4-butandiol und 2,2-Dimethyl-1,3-propandiol geeignet.

Erfindungsgemäß wird bei diesem Verfahren eine Amidosulfonsäure oder Alkylamidosulfonsäure als Katalysator in einer Konzentration von 0,001 bis 10, bevorzugt von 0,001 bis 5 mol%, bezogen auf 3-Methyl-2-butenal, eingesetzt.

Bei dem erfindungsgemäßen Verfahren zur Herstellung der Acetale der Formel (I) des Typs (a) setzt man 3-Methyl-2-butenal, Alkohole der Formel (IIa) oder Orthoester der Formel (III) bevorzugt in einem molaren Verhältnis (V1), gebildet aus der Menge an 3-Methyl-2-butenal und der Menge an von diesen Alkoholen und Orthoestern abgeleiteten Einheiten der Formel (IVa)

-OR (IVa)

in der R die gleiche Bedeutung wie in Formel (I) hat, ein das 0,5 :1 bis 0,01:1, besonders bevorzugt 0,4 bis 0,05 beträgt, wobei die Menge an Einheiten der Formel (IVa), die von den Orthoestern der Formel (III) abgeleitet sind, nur zu 2/3 berücksichtigt wird.

Zur Herstellung der Acetale des Typs (b) werden 3-Methyl-2-butenal und Alhohole der Formel IIb im allgemeinen in einem molaren Verhältnis (V2) von 0,02 : 1 bis 1 :1 einsetzt.

Im allgemeinen wird ein einheitliches Produkt der Formel (I) gewünscht, in dem der Rest R immer für die gleiche Kohlenwasserstoffgruppe steht und entsprechend werden dann die Alkohole der Formel (IIa) und die Orthoester der Formel (III) bzw. die Alkohole der Formel (IIb) so gewählt, daß der Rest R auch bei den Ausgangsprodukten für die gleiche Alkyl-, Alkenyl- bzw. Alkandiylgruppe steht.

Da die Acetalisierung von Aldehydgruppen mit Alkoholen eine Gleichgewichtsreaktion ist, bei der in den meisten Fällen das Gleichgewicht nicht gänzlich auf der Seite des Acetals liegt, ist es zur Optimierung der Ausbeute in vielen Fällen erforderlich, daß man das bei der Umsetzung von 3-Methyl-2-butenal mit den Alkoholen der Formel (IIa) oder (IIb) gebildete Wasser kontinuierlich aus der Reaktionsmischung zu entfernen.

Grundsätzlich existieren 2 verschiedene Möglichkeiten, um das Reaktionswasser aus der Reaktionsmischung zu entfernen.

Bei der ersten Methode wird die Umsetzung in Gegenwart eines inerten organischen Lösungsmittels durchführt, das mit Wasser ein Azeotrop bildet und mit Wasser bei Raumtemperatur so schlecht mischbar ist, daß nur solche überwiegend das organische Lösungsmittel enthaltende Mischungen existieren, die bei Raumtemperatur weniger Wasser enthalten als das Azeotrop, und mit diesem das Wasser abdestilliert wird. Üblicherweise wird anschließend das Azeotrop kondensiert, bevorzugt auf eine Temperatur unterhalb der Raumtemperatur abkühlt, die gebildete wässerige Phase abgetrennt und die organische Phase in die Reaktionsmischung zurückführt. Diese allgemein als azeotrope Destillation bekannte Methode und die hierfür geeigneten Lösungsmittel ("Wasserschlepper") sind beispielsweise in "Organikum, VEB Deutscher Verlag der Wissenschaften, Berlin 1981, 2. berichtigter Nachdruck der 15. Auflage, Kapitel 2.3.5 beschrieben.

Als Wasserschlepper kommen außerdem, falls eine Acetalisierung mit diesen Alkoholen beabsichtigt ist, Alkohole mit 5 bis 20 C-Atomen in Betracht, die man in einer Konzentration von 50 bis 99 Gew.-%, bezogen auf die Reaktionsmischung, verwendet.

Im allgemeinen verzichtet man auf den Einsatz der Orthoester der Formel (III), wenn das Wasser nach dem Verfahren der azeotropen Destillation entfernt wird.

Bei der Herstellung der Acetale des Typs (a) der Formel (I) kann man das gebildete Reaktionswasser nach einer weiteren Methode chemisch binden, indem man eine Mischung von Orthoester der Formel (III) und Alkoholen der Formel (IIa) einsetzt. Der Orthoester reagiert mit dem Wasser zum Monoester und setzt dabei 2 Äquivalente des Alkohols frei, der dann auch als Ausgangsprodukt für die Acetalisierung zur Verfügung steht. Der Orthoester wird deshalb günstigerweise in solchen Mengen eingesetzt, daß er sowohl Wasser zu binden vermag, das aus der Reaktion des 3-Methyl-2-butenals mit dem ursprünglich eingesetzten Alkohol stammt, als auch solches Wasser, das aus der Reaktion des 3-Methyl-2-butenals mit dem Alkohol, der wahrend der Reaktion aus dem Orthoester gebildet wurde.

Aus diesem Grund setzt man üblicherweise pro Mol Alkohol, der mit dem 3-Methyl-2-butenal reagieren kann, mindestens 0,5, bevorzugt 0,5 bis 2 mol Orthoester ein, mit der Maßgabe, daß die Menge an überschüssigem Alkohol, die die Menge an ursprünglich vorhandenem 3-Methyl-2-butenal um das Doppelte übersteigt und nach den Gesetzen der Stöchiometrie nicht mehr mit der Aldehydgruppe reagieren kann, dabei unberücksichtigt bleibt.

Die Reaktionstemperatur bedarf bei dieser Art der Umsetzung keiner exakten Kontrolle und kann im allgemeinen in einem Bereich von -20 bis 150°C, liegen.

Der bei der Reaktion anzuwendende Druck ist ebenfalls unkritisch und liegt üblicherweise bei 0,1 bis 1,5 bar.

Die Umsetzung ist beendet, wenn praktisch die Hauptmenge an 3-Methyl-2-butenal verbraucht ist. Im allgemeinen ist dies nach nach einer Reaktionszeit von 20 min bis 20 h der Fall.

Da Amidosulfonsäure und Alkylamidosulfonsäuren in der Reakcionsmischung löslich ist, läßt sich die Reaktionsmischung besonder einfach aufarbeiten, indem man eine für die Neutralisation der Amidosulfonsäure ausreichende Menge einer Base, bevorzugt eines Alkali- oder Erdalkalicarbonates, z.B. Kaliumcarbonat oder eines Amins zugibt und anschließend direkt destilliert.

### Experimenteller Teil

### Beispiel 1

76 g (2,37 mol) Methanol, 220 g (2,07 mol) Trimethylorthoformiat und 330 mg (3,4 mmol) Amidosulfonsäure wurden vorgelegt. Über einen Zeitraum von 2,5 Stunden wurden 125 g (1,49 mol) 3-Methyl-2-butenal zugetropft. Die Temperatur stieg infolge der exothermen Reaktion auf 33°C an. Nach weiteren 1,5 Stunden Rühren wurde die Reaktionsmischung gewogen (412,3 g) und mit 1 g K₂CO₃ versetzt. GC-analytisch wurde ein Gehalt von 43,7 Gew.% an 3-Methyl-2-butenal-dimethylacetal bestimmt. Die daraus berechnete Ausbeute betrug 93%.

### Beispiel 2

38 g (1,18 mol) Methanol, 171,7 g (1,62 mol) Trimethylorthoformiat und 30 mg (0,31 mmol) Amidosulfonsäure wurden vorgelegt. Innerhalb einer Stunde wurden 125 g (1,49 mol) 3-Methyl-2-butenal zugetropft. Die Temperatur stieg infolge der exothermen Reaktion auf 34°C an. Der Reaktionsansatz wurde weitere 3 Stunden gerührt, anschließend gewogen (330,4 g) und mit 1 g K₂CO₃ versetzt. GC-analytisch wurde ein Gehalt von 53,3 Gew.% an 3-Methyl-2-butenaldimethylacetal bestimmt. Die daraus berechnete Ausbeute betrug 91%.

### Beispiel 3

268,5 g (8,39 mol) Methanol, 1313,2 g (12,39 mol) Trimethylorthoformiat und 210 mg (2,17 mmol) Amidosulfonsäure wurden vorgelegt. Innerhalb einer Stunde wurden 875 g (10,42 mol) 3-Methyl-2-butenal zugetropft. Die Temperatur stieg infolge der exothermen Reaktion auf 45°C an. Der Reaktionsansatz wurde weitere 4 Stunden gerührt, anschließend gewogen (2401,7 g) und mit 17 g K₂CO₃ versetzt. GC-analytisch wurde ein Gehalt von 51,7 Gew.% an 3-Methyl-2-butenal-dimethylacetal gemessen. Die daraus berechnete Ausbeute betrug 91,7.%. Die Destillation der Reaktionsmischung ergab 1116,5 g 3-Methyl-2-butenal-dimethylacetal in einer Reinheit von 98,8% (8,49 mol). Die Ausbeute lag bei 81,5%.

### Beispiel 4

106,6 g (2,32 mol) Ethanol, 306 g (2,07 mol) Triethylorthoformiat und 330 mg (3,4 mmol) Amidosulfonsäure wurden vorgelegt. Innerhalb von 2,5 Stunden wurden 125 g (1,49 mol) 3-Methyl-2-butenal unter Eiskühlung zugetropft. Der Reaktionsansatz wurde weitere 1,5 Stunden gerührt, anschließend gewogen (535,7 g) und mit 10 g K₂CO₃ versetzt. GC-analytisch wurde ein Gehalt von 42,7 Gew.% an 3-Methyl-2-butenal-diethylacetal bestimmt. Die daraus berechnete Ausbeute betrug 97%. Die Destillation der Reaktionsmischung ergab 174,6 g 3-Methyl-2-butenal-diethylacetal in einer Reinheit von 98,6% (1,09 mol; 73,1% Ausbeute). Unter Berücksichtigung einer Mischfraktion von 145,5 g, die 21,8% des Acetals enthielt (0,2 mol), ergab sich eine isolierte Gesamtausbeute von 86,6%.

### Allgemeine Herstellvorschrift für Beispiele 5 bis 8 und Vergleichsbeispiele 1 bis 8

In 1,6 Äquivalenten Methanol wurde, wenn möglich, der in Tabelle 1 bzw. 2 angegebene Katalysator gelöst. Die erhaltene Lösung wurde zu einer Mischung von einem Äquivalent 3-Methyl-2-butenal und 1,4 Äquivalenten Trimethylorthoformiat getropft. Die Temperatur wurde bei exothermen Reaktionen durch Eiskühlung unterhalb von 30°C gehalten. War die Reaktionsgeschwindigkeit zu langsam, wurde zum Pückfluß erhitzt. In den Fällen, in denen der Katalysator nicht in Methanol löslich war, wurde eine Mischung von Katalysator und Methanol vorgelegt und 3-Methyl-2-butenal und Trimethylorthoformiat wurden zugetropft.

Die sonstigen Reaktionsbedingungen und die erzielten Ausbeuten können den Tabellen 1 und 2 entnommen werden.

**Tabelle 1:**

| Beispiele | | | | | | |
|---|---|---|---|---|---|---|
| Beispiele | Katalysator | mol% Katalysator | Temperatur [°C] | Reaktions―zeit [h] | GC―Analyse [in Fl.―%] | |
| | | | | | 3―Methyl―2-butenal | 3―Methyl―2―butenal-dimethyl-acetal |
| 5 | H₂NSO₃H | 4,6 | 32 | 1 | 0 | 50,76 |
| 6 | H₂NSO₃H | 2,3 | 10-12 | 2,5 | 0 | 51,29 |
| 7 | H₂NSO₃H | 0,23 | 31 | 3,5 | 0 | 60,31 |
| 8 | H₂NSO₃H | 0,23 | 33 | 3,5 | 0 | 61,62 |

**Tabelle 2:**

| Vergleichsbeispiele | | | | | | |
|---|---|---|---|---|---|---|
| Vergl. bsp. | Katalysator | mol% Katalysator | Temperatur [°C] | Heaktions―zeit [h] | GC―Analyse [in Fl.―%] | |
| | | | | | 3―Methyl―2―butenal | 3―Methyl―2―butenal-dimethylacetal |
| 1 | H₂SO₄ | 2,4 | 24-28 | 1 | 0,71 | 0,84 |
| 2 | (NH₄)₂SO₄ | 4,6 | 24-50 | 27 | 3,93 | 57,74 |
| 3 | (NH₄)₂SO₄ | 4,6 | 54 | 68 | 3,01 | 57,91 |
| 4 | HOAc | 4,6 | 50 | 24 | 34,95 | 16,50 |
| 5 | NH₄HSO₄ | 4,6 | 30 | 3 | 0,14 | 13,74 |
| 6 | HCOOH | 4,6 | 50 | 44 | 3,35 | 57,48 |
| 7 | NH₄Cl | 4,6 | 40 | 24 | 0,31 | 61,77 |
| 8 | NH₄HCO₂ | 4.6 | 40 | 18 | 33,6 | 2.28 |

## Patentansprüche

1. Verfahren zur Herstellung von Acetalen der Formel I 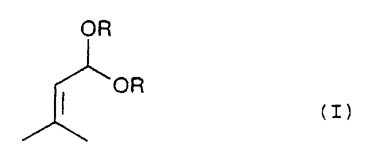 in der
a) R unabhängig voneinander für einen C₁- bis C₂₀-Alkyloder C₃- bis C₂₀-Alkenylrest stehen öder
b) die beiden Reste R gemeinsam die Glieder eines gegebenenfalls C₁- bis C₁₀-alkylsubstituierten 5 bis 7-gliedrigen cyclischen Acetals bilden,
indem man 3-Methyl-2-butenal in Gegenwart von Amidosulfonsäure, einer N-(C₁- bis C₈-Alkyl-)amidosulfonsäure oder einer N,N-Di(C₁- bis C₈-alkyl-)amidosultonsaure als Katalysator mit
im Fall (a)
a1) einem Alkohol der allgemeinen Formel IIa
R-OH (IIa)
in der R die unter (a) genannte Bedeutung hat,
a2) einem Orthoester der allgemeinen Formel (III)
HC-(OR)₃ (III)
in der R die unter (a) genannte Bedeutung hat oder
a3) einer Mischung aus einem Alkohol der allgemeinen Formel (IIa) und einem Orthoester der allgemeinen Formel (III),
und im Fall (b)
b) mit einem Alkohol der allgemeinen Formel IIb
HO-(CH₂)ₘ-OH (IIb)
in der m eine Zahl von 2 bis 4 bedeutet, und eine Methylengruppe im Alkohol IIb durch eine C₁-C₁₀-Alkylgruppe substituiert sein kann, umsetzt.

2. Verfahren nach Anspruch 1, **dadurch gekennzeichnet, daß** man Amidosulfonsäure, eine N-(C₁- bis C₈-Alkyl-)amidosulfonsäure oder eine N,N-Di(C₁- bis C₈-alkyl-)amidosulfonsäure in einer Konzentration von 0,001 bis 10 mol%, bezogen auf 3-Methyl-2-butenal, einsetzt.

3. Verfahren nach Anspruch 1 oder 2, **dadurch gekennzeichnet, daß** man das bei der Umsetzung von 3-Methyl-2-butenal mit den Alkoholen der Formel (IIa) oder (IIb) gebildete Wasser kontinuierlich aus der Reaktionsmischung entfernt.

4. Verfahren nach den Ansprüchen 1 bis 3, **dadurch gekennzeichnet, daß** man die Alkohole der Formel (IIa) und die Orthoester der Formel (III) in einem molaren Verhältnis von 0,01 : 1 bis 20 : 1 einsetzt.

5. Verfahren nach den Ansprüchen 1 bis 4, **dadurch gekennzeichnet, daß** man solche Mengen an 3-Methyl-2-butenal, Alhoholen der Formel IIa oder Orthoestern der Formel (III) einsetzt, daß das molare Verhältnis (V1), gebildet aus der Menge an 3-Methyl-2-butenal und der Menge an von diesen Alkoholen und Orthoestern abgeleiteten Einheiten der Formel (IVa)
-OR (IVa)
in der R die gleiche Bedeutung wie in Formel (I) hat, 0,5 : 1 bis 0,01 : 1 betrage, wobei die Menge an Einheiten der Formel (IVa), die von den Orthoestern der Formel (III) abgeleitet sind, nur zu 2/3 berücksichtigt wird.

6. Verfahren nach den Ansprüchen 1 bis 4, **dadurch gekennzeichnet, daß** man 3-Methyl-2-butenal und die Alhohole der Formel IIb in einem molaren Verhältnis (V2) von 0,02 : 1 bis 1 : 1 einsetzt.

7. Verfahren nach den Ansprüchen 1 bis 6, **dadurch gekennzeichnet, daß** man die Alkohole der Formel (IIa) und (IIb) sowie die Orthoester der Formel (III) so wählt, daß nur Verbindungen der Formel (I) gebildet werden, in denen die Reste R die gleiche Bedeutung haben.

8. Verfahren nach den Ansprüchen 1 bis 7, **dadurch gekennzeichnet, daß** man die Umsetzung in Gegenwart eines inerten organischen Lösungsmittels durchführt, das mit Wasser ein Azeotrop bildet und mit Wasser bei Raumtemperatur so schlecht mischbar ist, daß nur solche überwiegend das organische Lösungsmittel enthaltende Mischungen existieren, die bei Raumtemperatur weniger Wasser enthalten als das Azeotrop, und mit diesem das Wasser abdestilliert.

9. Verfahren nach Anspruch 7, **dadurch gekennzeichnet, daß** man, sofern R in der Verbindung der Formel (I) für einen Rest mit 5 bis 20 C-Atomen steht, als organisches Lösungsmittel den entprechenden Alkohol in einer Konzentration von 50-99 Gew.-%, bezogen auf die Reaktionsmischung, einsetzt.

10. Verfahren nach Anspruch 8 und 9, **dadurch gekennzeichnet, daß** man das Azeotrop kondensiert, auf eine Temperatur unterhalb der Raumtemperatur abkühlt, die gebildete wässerige Phase abtrennt und die organische Phase in die Reaktionsmischung zurückführt.

11. Verfahren nach den Ansprüchen 7 bis 10, **dadurch gekennzeichnet, daß** man auf den Einsatz von Orthoestern der Formel III verzichtet.

12. Verfahren nach den Anspruch 5, **dadurch gekennzeichnet, daß** man eine Mischung von Orthoester der Formel (III) und Alkohol der Formel (IIa) einsetzt, wobei man pro Mol Alkohol, der mit dem 3-Methyl-2-butenal reagieren kann, mindestens 0,5 mol Orthoester einsetzt, mit der Maßgabe, daß die Menge an Alkohol, die die Menge an ursprünglich vorhandenem 3-Methyl-2-butenal um das Doppelte übersteigt und nach den Gesetzen der Stöchiometrie nicht mehr mit der Aldehydgruppe reagieren kann, dabei unberücksichtigt bleibt.

## Claims

1. A process for preparing acetals of the formula I 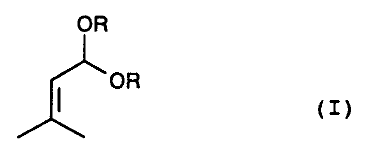 where
a) the Rs are, independently of one another, a C₁-C₂₀-alkyl or C₃-C₂₀-alkenyl radical or
b) the two R radicals together form the members of an unsubstituted or C₁-C₁₀-alkyl-substituted 5- to 7-membered cyclic acetal,
reacting 3-methyl-2-butenal in the presence of sulfamic acid, of a N-(C₁-C₈-alkyl)sulfamic acid or of a N,N-di(C₁-C₈-alkyl)sulfamic acid as catalyst with
in case (a)
a1) an alcohol of the formula IIa
R-OH (IIa)
where R has the meaning stated under (a),
a2) an orthoester of the formula (III)
HC-(OR)₃ (III)
where R has the meaning stated under (a) or
a3) a mixture of an alcohol of the formula (IIa) and an orthoester of the formula (III),
and in case (b)
b) with an alcohol of the formula IIb
HO-(CH₂)ₘ-OH (IIb)
where m is a number from 2 to 4, and a methylene group in the alcohol IIb may be substituted by a C₁-C₁₀-alkyl group.

2. A process as claimed in claim 1, wherein sulfamic acid, a N-(C₁-C₈-alkyl)sulfamic acid or a N-N-di(C₁-C₈-alkyl)sulfamic acid is employed in a concentration of from 0.001 to 10 mol% based on 3-methyl-2-butenal.

3. A process as claimed in claim 1 or 2, wherein the water formed in the reaction of 3-methyl-2-butenal with the alcohols of the formula (IIa) or (IIb) is removed continuously from the reaction mixture.

4. A process as claimed in any of claims 1 to 3, wherein the alcohols of the formula (IIa) and the orthoesters of the formula (III) are employed in a molar ratio of from 0.01 : 1 to 20 : 1.

5. A process as claimed in any of claims 1 to 4, wherein the amounts of 3-methyl-2-butenal, alcohols of the formula (IIa) or orthoesters of the formula (III) employed are such that the molar ratio (R1), formed from the amount of 3-methyl-2-butenal and the amount of units which are derived from these alcohols and orthoesters and are of the formula (IVa)
-OR (IVa)
where R has the same meaning as in formula (I),
is from 0.5:1 to 0.01:1, taking only 2/3 of the amount of units of the formula (IVa) derived from orthoesters of the formula (III) into account.

6. A process as claimed in any of claims 1 to 4, wherein 3-methyl-2-butenal and the alcohols of the formula IIb are employed in a molar ratio (R2) of from 0.02 : 1 to 1 : 1.

7. A process as claimed in any of claims 1 to 6, wherein the alcohols of the formula (IIa) and (IIb), and the orthoesters of the formula (III), are chosen so that there is formation only of compounds of the formula (I) where the R radicals have the same meaning.

8. A process as claimed in any of claims 1 to 7, wherein the reaction is carried out in the presence of an inert organic solvent which forms an azeotrope with water and is so poorly miscible with water at room temperature that the only mixtures predominantly containing the organic solvent which exist are those which contain less water at room temperature than does the azeotrope, and the water is distilled out with the latter.

9. A process as claimed in claim 7, wherein, when R in the compound of the formula (I) is a radical having 5 to 20 carbon atoms, the organic solvent employed is the corresponding alcohol in a concentration of 50-99% of the weight of the reaction mixture.

10. A process as claimed in claim 8 and 9, wherein the azeotrope is condensed and cooled to a temperature below room temperature, the aqueous phase which forms is separated off and the organic phase is returned to the reaction mixture.

11. A process as claimed in any of claims 7 to 10, wherein orthoesters of the formula III are not employed.

12. A process as claimed in claim 5, wherein a mixture of orthoester of the formula (III) and alcohol of the formula (IIa) is employed, and at least 0.5 mol of orthoester is employed per mole of alcohol able to react with 3-methyl-2-butenal, with the proviso that the amount of alcohol exceeding twice the amount of 3-methyl-2-butenal originally present, and unable to react with the aldehyde according to the laws of stoichiometry, is left out of account in this.

## Revendications

1. Procédé pour la préparation d'acétals de formule I 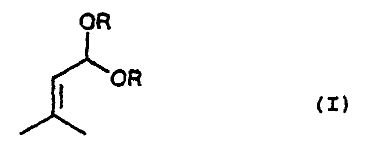 dans laquelle
a) R représentent indépendamment l'un de l'autre un reste alkyle en C₃ à C₂₀ ou alcényle en C₃ à C₂₀, ou
b) les deux restes R forment conjointement les chaînons d'un acétal cyclique constitué de 5 à 7 chaînons et étant éventuellement substitué par des groupes alkyle en C₁ à C₁₀,
en faisant réagir le 3-méthyl-2-buténal en présence de l'acide amidosulfonique, de l'acide N-(alkyle en C₁ à C₈)amidosulfonique ou de l'acide N,N-di(alkyle en C₁ à C₈)amidosulfonique en tant que catalyseur, et, dans- le cas (a)
a1) un alcool de formule générale IIa
R-OH (IIa)
dans laquelle R prend la signification précitée dans (a),
a2) un orthoester de formule générale (III)
HC-(OR)₃ (III)
dans laquelle R prend la signification précitée dans (a), ou
a3) un mélange de l'alcool de formule générale (IIa) et de l'orthoester de formule générale (III), et dans le cas (b)
b) un alcool de formule générale IIb
HO-(CH₂)ₘ-OH (IIb)
dans laquelle m représente un nombre compris entre 2 et 4 et dans laquelle un groupe méthylène de l'alcool IIb est éventuellement substitué par un groupe alkyle en C₁ à C₁₀.

2. Procédé selon la revendication 1, **caractérisé en ce que** l'on met en oeuvre l'acide amidosulfonique, l'acide N-(alkyle en C₁ à C₈)amidosulfonique ou l'acide N,N-di(alkyle en C₁ à C₈)amidosulfonique à une concentration comprise entre 0,001% en moles et 10% en moles, par rapport au 3-méthyl-2-buténal.

3. Procédé selon la revendication 1 ou 2, **caractérisé en ce que** l'on sépare en continu du mélange réactionnel l'eau formée lors de la réaction du 3-méthyl-2-buténal et des alcools de formule (IIa) ou (IIb).

4. Procédé selon les revendications 1 à 3, **caractérisé en ce que** l'on met en oeuvre les alcools de formule (IIa) et les orthoesters de formule (III) dans une proportion molaire comprise entre 0,01:1 et 20:1.

5. Procédé selon les revendications 1 à 4, **caractérisé en ce que** l'on met en oeuvre le 3-méthyl-2-buténal, l'alcool de formule IIa ou l'orthoester de formule (III) en quantités telles que la proportion molaire (V1) entre la quantité du 3-méthyl-2-buténal et la quantité des composés dérivés de cet alcool et de cet orthoester, composés dérivés de formule (IVa)
-OR (IVa)
dans laquelle R prend la signification précitée dans la formule (I), est comprise entre 0,5:1 et 0,01:1, seule 2/3 de la quantité du composé de formule (IVa) qui est dérivée de l'orthoester de formule (III) compte.

6. Procédé selon les revendications 1 à 4, **caractérisé en ce que** l'on met en oeuvre le 3-méthyl-2-buténal et l'alcool de formule IIb dans une proportion molaire (V2) comprise entre 0,02:1 et 1:1.

7. Procédé selon les revendications 1 à 6, **caractérisé en ce que** l'on choisit les alcools de formule (IIa) et (IIb) ainsi que l'orthoester de formule (III) de sorte que l'on obtient uniquement des composés de formule (I) dans laquelle les restes R sont identiques.

8. Procédé selon les revendications 1 à 7, **caractérisé en ce que** l'on procède à la réaction en présence d'un solvant organique inerte qui forme avec l'eau un azéotrope et qui présente, à température ambiante, une miscibilité avec l'eau tellement médiocre que les mélanges existants et contenant pour l'essentiel le solvant organique, contiennent, à température ambiante, moins d'eau que l'azéotrope, et à l'aide duquel on sépare l'eau au moyen d'une distillation.

9. Procédé selon la revendication 7, **caractérisé en ce que** l'on met en oeuvre en tant que solvant organique, dans le cas où R dans le composé de formule (I) représente un reste présentant 5 à 20 atomes de C, l'alcool correspondant à une concentration comprise entre 50% et 99% en poids par rapport au mélange réactionnel.

10. Procédé selon les revendications 8 et 9, **caractérisé en ce que** l'on soumet l'azéotrope à une condensation, qu'on le refroidit à une température inférieure à la température ambiante, que l'on sépare la phase aqueuse ainsi formée et que l'on recycle la phase organique dans le mélange réactionnel.

11. Procédé selon les revendications 7 à 10, **caractérisé en ce que** l'on renonce à la mise en oeuvre d'orthoesters de formule III.

12. Procédé selon la revendication 5, **caractérisé en ce que** l'on met en oeuvre un mélange constituée de l'orthoester de formule (III) et un alcool de formule (IIa), en employant, par mole d'alcool étant apte à réagir avec le 3-méthyl-2-buténal, au moins 0,5 mole d'orthoester, à la condition qu'en ce cas on ne tient pas compte de la quantité d'alcool qui est le double de la quantité initiale du 3-méthyl-2-buténal et qui, d'après les lois de la stoechiométrie, ne peut plus réagir avec le groupe aldéhyde.
